(19) Europäisches Patentamt
European Patent Office
Office européen des brevets

(11) Veröffentlichungsnummer: **0 006 192**
**B1**

(12) # EUROPÄISCHE PATENTSCHRIFT

(45) Veröffentlichungstag der Patentschrift: **25.02.81**

(51) Int. Cl.³: **C 12 M 1/00**

(21) Anmeldenummer: **79101806.2**

(22) Anmeldetag: **07.06.79**

(54) **Vorrichtung und Verfahren für mikrobiologische Arbeiten.**

(30) Priorität: **12.06.78 DE 2825636**

(43) Veröffentlichungstag der Anmeldung: **09.01.80 Patentblatt 80/1**

(45) Bekanntmachung des Hinweises auf die Patenterteilung: **25.02.81 Patentblatt 81/8**

(84) Benannte Vertragsstaaten: **AT BE CH DE FR GB IT LU NL SE**

(56) Entgegenhaltungen:
**DE-A-2 320 946**
**FR-A-2 374 353**
**US-A-2 672 431**
**US-A-2 672 432**

(73) Patentinhaber: **BOEHRINGER MANNHEIM GMBH,** ***Sandhofer Strasse*** **112-132** ***Postfach*** **31 01 20, D-6800 Mannheim 31-Waldhof (DE)**

(72) Erfinder: **Wielinger, Hans, Dr., Kronbergerstrasse 14, D-6800 Mannheim 31 (DE)**
Erfinder: **Rittersdorf, Walter, Dr., Kasseler Strasse 6, D-6800 Mannheim 31 (DE)**
Erfinder: **Bleisteiner, Manfred, Feldstrasse 93, D-6800 Mannheim 31 (DE)**
Erfinder: **Zimmermann, Gerd, Lärchenweg 6a, D-6940 Weinheim (DE)**
Erfinder: **Werner, Wolfgang, Dr., Meissener Weg 39, D-6800 Mannheim 42 (DE)**
Erfinder: **Vömel, Wolfgang, Dr., Maximilianstrasse 2a, D-6800 Mannheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

**EP 0 006 192 B1**

BUNDESDRUCKEREI BERLIN

# Vorrichtung und Verfahren für mikrobiologische Arbeiten

Die Erfindung betrifft eine Vorrichtung für mikrobiologische Arbeiten, bestehend aus einem Nährkarton und einem darüberliegenden Film aus Kunststoffdispersion. Nährkarton und Dispersionsfilm sind zu einer Testeinheit zusammengefaßt.

Die Untersuchung bestimmter Körperflüssigkeiten auf das Vorhandensein von Mikroorganismen, wie der Nachweis von Bakterien und Pilzen in Urinen, ergibt für den Arzt, den Mikrobiologen usw. wertvolle Hinweise für die Diagnose bestimmter Erkrankungen. Zu diesem Zweck wurden innerhalb der letzten Jahre zahlreiche Prüfmittel zum Nachweis von Bakterien und Pilzen entwickelt.

Derartige Nachweismittel finden aber auch Verwendung zur Untersuchung von Arzneimitteln, Nahrungsmitteln, Wasser oder Schwimmbecken, wo bestimmte Arten von Mikroorganismen schädlich sind oder zu Gesundheitsgefährdungen führen können.

Die klassischen Verfahren zum Anzüchten und zum Nachweis von Mikroorganismen beruhen darauf, daß man verschiedene ausgewählte Nährstoffe und gegebenenfalls Selektivhemmstoffe zusammen mit Agar-Agar zu einem Nährmedium verarbeitet. So wichtig die Nährmedien für spezielle Fragestellungen sind, so weisen sie für die Routine doch Nachteile auf, speziell wenn es gilt, kostensparend größere Anzahlen von Proben zu untersuchen. Die Nährmedien müssen relativ umständlich aus einem trockenen Pulver für den Eigenbedarf vom Anwender selbst hergestellt werden. Es gibt auch gebrauchsfertige Nährböden im Handel, die in angequollener Form zur Verfügung stehen. Diese gebrauchsfertigen Nähragars trocknen aber leicht aus, sind dann nicht mehr reversibel befeuchtbar und müssen daher in wasserdampfdichten Verpackungen gelagert werden, die zudem noch ein großes Volumen einnehmen.

In neuerer Zeit sind speziell für Lebensmittel- und Wasser-Untersuchungen mikrobiologische Nachweisverfahren beschrieben, bei denen Nährkartons mit Membranfiltern bedeckt werden. Dazu wird die zu untersuchende Flüssigkeit durch ein Membranfilter filtriert, das Membranfilter auf einen mit sterilem Wasser befeuchteten Nährkarton oder einen mit steriler Nährlösung getränkten Filterkarton gelegt und diese Einheit in einer Petrischale bebrütet. Die auf dem Membranfilter zurückgehaltenen Mikroorganismen wachsen während der Bebrütungszeit und werden als Kolonien sichtbar.

Dieses Verfahren ist nur dann praktikabel, wenn Keime in sehr geringer Konzentration nachgewiesen werden sollen und deshalb vor dem Nachweis über das Bebrüten, angereichert werden müssen. Membranfilter und Nährkarton werden lose geliefert und bedürfen wegen der Sprödigkeit des Membranmaterials besonders sorgfältiger Behandlung. Einen Fortschritt bringt eine Verbesserung, die Membranfilter und Nährkarton in einem speziellen Rahmen zu einer Einheit vereint (DAS 2 115 674). Dieses Verfahren stellt zwar eine Vereinfachung in der Handhabung dar, die Herstellung der Einheit Membranfilter, Nährkarton in einem speziellen Rahmen ist jedoch aufwendig und teuer.

Die beschriebenen Filterverfahren sind für medizinisch diagnostische Fragestellungen nicht geeignet, da eine Konzentrierung der Keime auf der Filteroberfläche nicht erforderlich, im Gegenteil, wegen der Notwendigkeit hohe Keimzahlen, vorzugsweise im Bereich von $10^3 - 10^8$ Keime/ml Körperflüssigkeit zu unterscheiden, von großem Nachteil ist.

Des weiteren sind Verfahren bekannt, bei denen auf Nährkartons eine mikroporöse Membran erzeugt wird und zwar dergestalt, daß das Membranmaterial teilweise in den Karton eindringt (DOS 2 320 946). Dieses Verfahren hat den Nachteil, daß die Membranoberfläche, auf der die Keime wachsen sollen, dieselbe Rauhigkeit/Unebenheit wie der Nährkarton aufweist. Die Keime sind bei einem solchen Test wegen der Unebenheiten der Oberfläche ohne zusätzliche Hilfsmaßnahmen nach dem Bebrüten nicht oder äußerst schlecht sichtbar. Sie müssen durch Anfärben mit Farbstoffen sichtbar gemacht werden. Die Keime zeigen sich daher dem Betrachter in völlig anderer Art und Weise wie bei dem klassischen Nachweisverfahren auf Agar. Eine visuelle Differenzierung der Spezies ist nicht möglich. Die Kolonien, die mehr oder minder stark in den Nährkarton eindringen, können nicht gezielt abgeimpft werden. Dieses Abimpfen ist jedoch für eine Keimdifferenzierung und die Untersuchung des Resistenzverhaltens unbedingt notwendig.

Ein anderes Verfahren ist bekannt, das im wesentlichen eine Weiterentwicklung des oben beschriebenen Verfahrens darstellt. Es hat den Zweck, den Nachteil dieses Verfahrens zu beheben. Dieser in der DOS 2 320 943 beschriebene Test wurde dahingehend verbessert, daß statt der Membranschicht oder über die Membranschicht eine Gelschicht angebracht wird und daß die Gelschicht und der Nährkarton ineinander übergehen. Dabei müssen zweckmäßigerweise in die Gelschicht auch Nährstoffe eingearbeitet werden. Solche Gelschichten sind erfahrungsgemäß auf Papieroberflächen nicht mit derselben Gleichmäßigkeit und Glätte aufzubringen, wie dies für gequollene Agarschichten möglich ist. Dieses Verfahren weist daher ähnliche Nachteile auf, wie ein Verfahren gemäß DOS 2 320 946.

Die Aufgabenstellung der vorliegenden Erfindung war daher: Entwicklung eines Bakterien-Trockennährbodens, der beim Eintauchen in die zu untersuchende, keimhaltige Lösung entsprechend rasch Wasser aufnimmt und dessen Oberfläche im feuchten Zustand so glatt und gleichmäßig ist, wie die der klassischen Agarmedien. Auf der Oberfläche müssen die Keime beim Eintauchen in keimhaltige Lösungen in einer ihrer Konzentration äquivalenten Menge haften bleiben. Die Keime müssen innerhalb der üblichen Bebrütungszeit (12–24 Std.) Kolonien bilden, wie dies bei den klassischen Agarnährmedien der Fall ist. Mit diesen Trockennährböden müssen also die Keimzahl und die Keimspezies in gleicher Art und Weise ermittelt werden können, wie bei den klassischen Verfahren. Es muß auch möglich sein, das Resistenzverhalten der Keime zu testen.

Zur Lösung dieser Aufgabenstellung mußten spezielle Abdeckschichten zum Belegen von Nährkartons entwickelt werden, die die Nachteile der oben geschilderten Verfahren nicht aufweisen. Es wurde überraschenderweise gefunden, daß Filme auf Basis von Kunststoffdispersionen eine befriedigende Lösung des Problems ermöglichen. Dazu wurden Kunststoffdispersionen mit »inneren Öffnern« versehen, die dem aus der Dispersion hergestellten Film folgende Eigenschaften verleihen:

Der Film bleibt undurchlässig für Mikroorganismen.
Der Film ist durchlässig für Bakteriennährstoffe und Substanzen, die das Keimwachstum selektiv oder generell hemmen oder unterbinden.
Die Oberfläche des Films ist glatt.
An der Filmoberfläche werden die Keime entsprechend ihrer Konzentration im zu untersuchenden Medium festgehalten.

Als Dispersionsmaterial sind prinzipell alle wäßrigen Kunststoffdispersionen geeignet mit der Einschränkung, daß die aus diesen Dispersionen gebildeten Filme das Bakterienwachstum nicht negativ beeinflussen dürfen. Es können beispielsweise folgende Dispersionen eingesetzt werden: Homopolymere aus Vinylacetat oder Acrylsäureestern, Copolymere aus Vinylpropionat und Vinylacetat, Vinylpropionat und Vinylchlorid, Vinylacetat und Maleinsäureestern, Acrylsäureestern mit Acrylnitril und Vinylpropionat, Butadien oder Styrol.

Als Öffner sind prinzipiell alle wasserlöslichen bzw. wasserquellbaren, Verbindungen zu verwenden, die das Keimwachstum nicht negativ beeinflussen, sich im angefeuchteten Film nur soweit lösen bzw. anquellen, daß die Struktur des Films nicht so zerstört wird, daß der Film zerfällt oder so große Löcher entstehen, daß die Keime durchdringen können. Werden sie verstoffwechselt, so darf durch den Vorgang der Verstoffwechselung den Keimen nicht die Möglichkeit gegeben werden, durch den Film durchzudringen oder die Struktur desselben so zu zerstören, daß die Kolonien für den Betrachter nicht mehr eindeutig sichtbar werden.

Es sind selbstverständlich auch Gemische von Substanzen, die Öffnereigenschaften haben, verwendbar. Bei speziellen Fragestellungen ist der Einsatz von mehreren Öffnern in einem Film sogar von Vorteil.

Als Öffner können vorzugsweise folgende makromolekularen Substanzen eingesetzt werden: Polyäthylenglykole, Polyäthylenoxide, Polyvinylpyrrolidon, Polyvinylalkohole, teilverseifte Polyvinylester, Mischpolymerisate aus Vinylpyrrolidon und Vinylestern, Cellulosederivate, wie Hydroxyalkylcellulosen. Weiterhin können auch niedermolekulare Stoffe, wie Zucker und Nährsalze oder Gemische solcher makro- und mikromolekularen Stoffe verwendet werden.

Bei der Zusammensetzung der Formulierung der Filmrohmasse ist der Zusatz von Netzmittel zweckmäßig. Dadurch werden sowohl die Eigenschaften des Films verbessert, als auch das Wachstum bestimmter Spezies selektiv beeinflußt und ein nicht-gewünschtes Verhalten der Keime während der Bebrütungsphase, wie das Schwärmen des Proteus, unterbunden. Als Netzmittel sind alle in der Mikrobiologie üblicherweise verwendeten Substanzen, wie Natriumdodecylsulfat, Natriumheptadecylsulfat, N-Cetyl-N,N,N-trimethylammoniumbromid oder Polyoxyäthylensorbitanmonooleat anwendbar.

Da das Wachstum der Keime auch maßgeblich vom pH-Wert des Mediums auf bzw. in dem sie wachsen abhängig ist, stellt man mit Hilfe von üblichen Puffern (z. B. Sörensen-Puffer) den pH-Wert der Filmmasse auf den gewünschten Bereich ein. Die Salzkonzentration wird dabei so gewählt, daß die osmolaren Verhältnisse den Anforderungen für ein befriedigendes Keimwachstum entsprechen.

Zusätzlich zu den Öffnern (makromolekulare Verbindungen), Netzmitteln und Puffern, können in den Film noch Selektivhemmstoffe oder Antibiotika eingearbeitet werden, so daß über eine solche Formulierung das Wachstum von Keimen selektiv gehemmt werden, bzw. die minimale Hemmkonzentration von Antibiotika bestimmt werden kann.

Derartige Filme werden in an sich bekannter Weise wie folgt hergestellt:

Die Kunststoffdispersion wird mit einer Lösung des Öffners homogen verrührt. In diesem Gemisch werden die anderen Bestandteile, wie Netzmittel, Puffer, Hemmstoffe eingerührt. Bei diesen Filmrohmassen können die Mengenverhältnisse der Dispersionspolymeren zu den Öffnern von 50 : 1 bis 1 : 5 schwanken. Der Zusatz an Lösungsmittel richtet sich nach der gewünschten Viskosität der Masse, die je nach Verfahren zur Weiterverarbeitung sehr unterschiedlich sein kann. Die Konzentration der restlichen Zusatzstoffe richtet sich nach den Anforderungen, die gestellt werden, sie können 0 bis 15%, vorzugsweise 1–5% der Kunststoffabdeckschicht betragen.

Die Filme selbst werden in üblicher Weise dadurch hergestellt, daß man die Filmrohmasse in einer Schichtdicke von 10–1000 μ auf einem Untergrund, mit dem sich die Filmrohmasse nicht zu fest verbindet, nach bekannten Verfahren aufstreicht, aufrakelt oder aufspritzt. Nach dem Trocknen oder während des Trocknungsvorgangs wird der Film vom Untergrund angezogen oder abgehoben und anschließend weiterverarbeitet.

Um Filme gegen Dehnungen in die Längs- und Querrichtung zu stabilisieren, kann man sie so herstellen, daß man die Filmrohmasse auf einen inneren Träger, wie Gewebe und Gewirke, welcher auf einem inerten Untergrund liegt, in der o. a. Art und Weise aufbringt. Bevorzugte Träger sind handelsübliche Kunststoffnetze aus Polyamid, Polyester und Polyethylen, die z. B. unter dem Namen Müllergaze bekannt sind und beispielsweise eine Dicke von ca. 10–200 μ besitzen.

Solche gestützten Filme können auch dadurch hergestellt werden, daß man die inneren Träger mit der Filmrohmasse imprägniert oder beschichtet und gegebenenfalls durch anschließendes Abstreifen die Filmdicke einstellt. Werden solche Filme mit an sich bekanntem Nährkarton zu Testsystemen vereinigt, so erhält man einen Test, der den zuvor gestellten Anforderungen voll entspricht.

Nährkartons werden in bekannter Weise so hergestellt, daß Filterpapiere oder entsprechende andere saugfähige Trägerschichten mit Lösungen von in der Mikrobiologie üblichen Nährstoffgemischen getränkt und anschließend getrocknet werden. Auf diese Weise können eine Vielzahl von Nährkartons mit verschiedenen Eigenschaften hergestellt werden. Es sind dies z. B.: Nährmedium nach Mac Konkey, CLED-Nährmedium, Nährmedium nach Slanetz und Bartley, Chinablau-Lactose-Nährmedium, Endo-Nährmedium, Wismut-Sulfit-Nährmedium nach Wilson-Blair, Cetrimid-Nährmedium, CaSo-Nährmedium, Nährmedium nach Müller-Hinton und Sabouraud.

Der gebrauchsfertige Test kann verschieden aufgebaut sein. Die Modelle, die sich am besten bewährt haben, seien nun aufgeführt.

Ein freitragender Film oder ein Film, der durch einen inneren Träger stabilisiert ist, wird auf einen Nährkarton gelegt. Zum Nachweis von Mikroorganismen taucht man den Nährkarton und den Film in die zu untersuchende, keimhaltige Lösung. Beide werden übereinanderliegend in eine Petrischale gebracht und 12–24 Std. bebrütet. Die nachzuweisenden Keime zeigen sich in Form von Kolonien in bekannter Art und Weise.

Will man die Keimzahl quantifizieren, so ist es zweckmäßig, den Film so herzustellen, daß man als inneren Träger ein gewebtes Netz nimmt, bei dem in bestimmten Abständen, z. B.: 0,5 cm, die Fäden anders gefärbt sind. Taucht man einen solchen Film gemeinsam mit dem Nährkarton in eine keimhaltige Flüssigkeit und bebrütet anschließend, so kann man über die Anzahl von Kolonien pro Flächeninhalt die Keimzahl pro Volumen-Einheit sehr leicht ermitteln. Bei einer besonders einfach herzustellenden Vorrichtung legt man den Nährkarton auf eine steife Folie, legt ein etwas breites Stück des oben beschriebenen Testfilms darüber und schweißt oder klebt die überstehenden Teile des Films so auf die Folie, daß der Nährkarton in der zwischen Folie und Film entstandenen Tasche fest eingeklemmt wird. Dieser Teststreifen, der zweckmäßigerweise eine Breite von 0,5–5 cm hat, bei dem das Testfeld vorzugsweise quadratisch ist, wird für den Verbraucher wie folgt vorbereitet: Er wird zwischen Folien oder kaschierten Papieren eingesiegelt oder eingeklebt und nach üblichen Verfahren sterilisiert. So erhält man einen gebrauchsfertigen Trocken-Nährboden in einer äußerst platzsparenden Aufmachung. Der Nährboden ist außerdem noch wesentlich länger haltbar als Nähragars, da er nicht vertrocknen kann und die empfindlichen Nährstoffe in trocknem Zustand deutlich stabiler sind als in gequollenen Agars. Vor dem Gebrauch wird der Test aus der Folienverpakkung entnommen. Zum Nachweis von Keimen, z. B. im Harn, wird der Test in frischen, sauber aufgefangenen Mittelstrahlurin getaucht, in ein Bebrütungsgefäß gebracht und bebrütet. Gegebenenfalls kann auch die Verpackung als Bebrütungsgefäß dienen. Anhand von Vergleichstafeln können die Keimzahlen pro ml Harn ermittelt werden. Zweckmäßig ist es, nicht nur ein Universal-, sondern zusätzlich mehrere Selektiv-Nährmedien und/oder Elektiv-Nährmedien zu einer Testeinheit zu vereinigen.

Zur Erstellung eines Antibiogramms nimmt man eine solche Vorrichtung, die beispielsweise einen Universal-Nährkarton (z. B. Nährmedium nach Müller-Hinton) enthält, befeuchtet diese mit sterilem Wasser, verstreicht auf dieser mit dem Spatel die Aufschlämmung der zu testenden Keime und setzt Antibiotikablättchen, beispielsweise nach DIN 58 940, auf. Nach dem Bebrüten wertet man in üblicher Art und Weise aus.

Eine weitere Möglichkeit das Resistenzverhalten von Keimen zu untersuchen, ist die Bestimmung der minimalen Hemmkonzentration (MHK). Dazu wird folgendermaßen verfahren:

Man arbeitet die Antikiotika in den gewünschten Konzentrationen direkt in die Filmmasse ein und stellt aus den daraus hergestellten Filmen Teststreifen her. Dazu legt man einen Nährkarton mit einem Universal-Nährmedium auf eine Folie und siegelt den entsprechenden antibiotikahaltigen Film wie oben beschrieben darüber. Gegebenenfalls können die Antibiotika auch in den Nährkarton eingearbeitet sein.

Einige Antiotika sind nur in einem relativ engen pH-Bereich über längere Zeit stabil, einem pH-Bereich, der dem pH-Optimum des Keimwachstums nicht entspricht. Zur Bestimmung der MHK solcher Substanzen nach dem erfindungsgemäßen Verfahren werden die Filme wie folgt hergestellt und weiter wie oben beschrieben zu Teststreifen verarbeitet.

Man bringt auf einen der oben beschriebenen antibiotikafreien Film einen zweiten Film auf, der das gewünschte Antibiotikum enthält. Die Pufferkapazität des zweiten Filmes wird so eingestellt, daß beim Eintauchen in das zu untersuchende, wäßrige Probengut der Puffer des ersten Films bzw. des Nährmediums die Einstellung des für das Keimwachstum optimalen pH-Wertes gewährleistet. Der zweite Film kann entweder aus der gleichen Filmrohmasse hergestellt werden, wie der erste Film, mit dem Unterschied, daß er einen anderen, dem Antibiotikum entsprechenden pH-Wert haben muß. Es ist aber nicht notwendig, daß Kunststoffdispersion und Öffner in beiden Filmen identisch sind.

Man kann aber auch so vorgehen, daß man den zweiten Film in dem das Antibiotikum eingearbeitet ist, aus einem für das Keimwachstum weitgehend inerten, wasserlöslichen oder wasserquellbaren Filmbildner herstellt. Als Filmbildner werden dazu vorzugsweise dieselben Substanzen verwendet, die auch als Öffner eingesetzt werden können.

Ferner können der antibiotikahaltige Film und der Nährkarton unterschiedliche pH-Werte aufweisen, so daß der Puffer des Nährkartons erst nach dem Befeuchten auch auf der Oberfläche des Films den für das Keimwachstum optimalen pH-Wert einstellt.

Die Maßnahme, Antibiotika direkt in den Film bzw. den Nährkarton einzuarbeiten und diese zu einer Testeinheit zur Bestimmung der MHK zusammenzufügen, hat folgende Vorteile: Es ist nicht mehr notwendig, Antibiotikablättchen auf eine Agarplatte aufzulegen. Man kann ohne den bisher unumgängigen Zwischenschritt der Keimanzüchtung, direkt aus dem Probengut das Resistenzverhalten der Keime testen. Man erspart sich durch den neuen Weg das bisher zur Bestimmung der MHK notwendige, komplizierte Verfahren, die Antibiotika für jede Untersuchungsreihe von Neuem in Nährbouillons einzuwiegen.

Schließlich ist die Beurteilung des gehemmten Keimwachstums wesentlich leichter: Man muß nicht mehr den Trübungsgrad der Nährbouillon beurteilen, sondern kann auf der Filmoberfläche direkt das Keimwachstum bewerten und klassifizieren.

Die Möglichkeiten, die die vorliegende Erfindung bietet, sollen durch die folgenden Beispiele näher erklärt werden:

## Beispiel 1
## Herstellung von Filmen

Zur Herstellung geeigneter Filme können verschiedene Kunststoffdispersionen und Öffner Verwendung finden. Beide werden zusammen mit Hilfsstoffen zur Filmrohmasse verrührt. (Öffner sind wasserlösliche, relativ gering anquellende, hochpolymere Substanzen.)

Es wird gezeigt, welche Vielfalt von Kombinationsmöglichkeiten der Rohstoffe es erlaubt, brauchbare Filme herzustellen.

Die Öffner werden in Wasser unter Rühren gelöst, dazu kommt die Kunststoffdispersion, in den in den folgenden Tabellen angegebenen Mengen. Diese Mischung wird homogen verrührt. Danach wird 1 g Netzmittel zugegeben. Die Wahl des Netzmittels wird entsprechend der Keime, die nachgewiesen werden sollen, getroffen, z. B.: Natriumdodecylsulfat zur Hemmung der gramnegativen Flora, Polyoxyäthylensorbitanoleat als Zusatz beim Lactobacillen-Nachweis und zur Hemmung des Schwärmens von Keimen, Natriumheptadecylsulfat zur Hemmung der grampositiven Flora und bei Medien zur Anreicherung von coliformen Keimen, N-Cetyl-N,N,N-trimethylammoniumbromid zur selektiven Anzüchtung von Pseudomonas. Zusätzlich wird noch 1 ml einer gepufferten Kochsalzlösung (Zusammensetzung: 92 g $Na_2HPO_4 \cdot 2\,H_2O$, 21,1 g $KH_2PO_4$, 7,65 g NaCl in 1000 ml $H_2O$) zugefügt und mit 0,1 n Natronlauge auf einen pH von 6,9—7,5 eingestellt.

Tabellarische Aufstellung der Dispersions-, Öffner- und Wasser-Mengen

| | | | |
|---|---|---|---|
| Dispersion: | | | |
| Homopolymeres aus Acrylsäureester | 60 g | 50 g | 50 g |
| Öffner: | | | |
| Hydroxypropylmethylcellulose | 4 g | | |
| Copolymeres aus Äthylenglykol und Äthylenoxyd | | 18 g | |
| Polyvinylpyrrolidon | | | 24 g |
| Wasser | 30 ml | 26 ml | 31 ml |

Tabellarische Aufstellung der Dispersions-, Öffner- und Wasser-Mengen

| | | | | | | | | | | |
|---|---|---|---|---|---|---|---|---|---|---|
| Dispersion: | | | | | | | | | | |
| Copolymeres aus Vinylacetat und Vinylpropionat | 62 g | 54 g | 45 g | 50 g | 90 g | 50 g | 90 g | 50 g | 48 g | 48 g |
| Öffner: | | | | | | | | | | |
| Polyäthylenoxid | | 9 g | | | | | | | 4 g | 4 g |
| Polyvinylpyrrolidon | 16 g | | | | | | | | 14 g | |
| Copolymeres aus Äthylenoxid und Äthylenglykol | | | 22 g | | | | | | | |
| Copolymeres aus Vinylacetat und Vinylpyrrolidon | | | | 11 g | | | | | | 5 g |
| Polyvinylalkohol | | | | | 5 g | 5 g | | | | |
| Teilverseiftes Polyvinylacetat | | | | | | | 5 g | 5 g | | |
| Wasser | 22 ml | 36 ml | 32 ml | 33 ml | 13 ml | 13 ml | 50 ml | 50 ml | 33 ml | 27 ml |

| | | | | |
|---|---|---|---|---|
| Dispersion: | | | | |
| Copolymeres aus Butadien und Styrol | 52 g | 74 g | 62g | 62 g |
| Öffner: | | | | |
| Polyvinylpyrrolidon | 16 g | 16 g | | |
| Copolymeres aus Äthylenglykol und Äthylenoxid | | | 16 g | |
| Copolymeres aus Vinylacetat und Vinylpyrrolidon | | | | 16 g |
| Wasser | 22 ml | 22 ml | 22 ml | 47 ml |

| | | |
|---|---|---|
| Dispersion: | | |
| Homopolymeres aus Vinylacetat | 62 g | 62 g |
| Öffner: | | |
| Polyvinylpyrrolidon | 20 g | |
| Copolymeres aus Vinylacetat und Vinylpyrrolidon | | 16 g |
| Wasser | 26 ml | 47 ml |

| | | | |
|---|---|---|---|
| Dispersion: | | | |
| Copolymeres aus Acrylnitril, Acrylsäureester und Vinylpropionat | 78 g | 92 g | 78 g |

Tabellarische Aufstellung der Dispersions-, Offner- und Wasser-Mengen

| | | | |
|---|---|---|---|
| Öffner: | | | |
| Polyvinylpyrrolidon | 16 g | 16 g | |
| Copolymeres aus Äthylenglykol und Äthylenoxid | | | 16 g |
| Wasser | 22 ml | 22 ml | 23 ml |
| Dispersion: | | | |
| Copolymeres aus Maleinsäureester und Vinylacetat | 58 g | 60 g | 60 g |
| Öffner: | | | |
| Polyvinylpyrrolidon | 13 g | | |
| Polyäthylenoxid | | 16 g | |
| Copolymeres aus Vinylacetat und Vinylpyrrolidon | | | 16 g |
| Wasser | 18 ml | 62 ml | 47 ml |
| Dispersion: | | | |
| Homopolymeres aus Vinylchlorid | 62 g | | |
| Öffner: | | | |
| Polyäthylenoxid | 16 g | | |
| Wasser | 62 ml | | |
| Dispersion: | | | |
| Copolymeres aus Vinylchlorid und Vinylpropionat | 52 g | | |
| Öffner: | | | |
| Polyvinylpyrrolidon | 15,7 g | | |
| Wasser | 22 ml | | |

Die Filmrohmasse wird in an sich bekannter Weise auf eine inerte Unterlage mit einer Schichtdicke von 300 μ aufgerakelt und bei 50° —70° C getrocknet. Nach dem Trocknen wird der Film in die gewünschte Größe geschnitten und weiterverarbeitet.

Beispiel 2
Herstellung eines Teststreifens zur Bestimmung der Keimzahl im Harn und zur groben Differenzierung der Keime

62 g einer Dispersion aus einem Copolymerisat von Vinylacetat und Vinylpropionat werden mit einer Lösung von 16 g Polyvinylpyrrolidon in 22 ml Wasser verrührt. Dann wird wie in Beispiel 1 beschrieben 1 ml einer gepufferten physiologischen Kochsalzlösung und 1 ml Polyoxyäthylensorbitanmonoelat dazugerührt und mit Natronlauge auf einen pH von 7,3 eingestellt.

Auf eine Polyäthylenfolie wird ein Nylonnetz von einer Dicke von 50 µ gelegt, darauf wird die Filmmasse mit einer Schichtdicke von 350 µ aufgerakelt. Nach dem Trocknen bei 60°C wird der nun erhaltene gestützte Film von der Folie abgezogen und in Bahnen von 6 cm Breite geschnitten.

Auf eine 200 µ dicke Folie der Größe 2 x 12 cm werden nebeneinander zwei Nährkartons der Größe 2 x 2 cm mit Ca – So-Medium und Mac-Conkey-Medium gelegt. Über die beiden Nährkartons wird der Film gelegt und durch Verkleben an der Folie angeheftet (siehe Abb. 1 und 2). Nach dem eben beschriebenen Verfahren wird ein zweiter Teststreifen hergestellt, bei dem als Nährmedien Nährkartons mit Pseudomonas-Elektiv und Enterokokken-Selektiv-Medien eingesetzt werden. Die beiden Teststreifen werden durch Verkleben zu einem Teststreifen vereinigt (siehe Abb. 1 und 2). Um den Teststreifen gegen Kontamination und Feuchtigkeit zu schützen, wird er in z. B. mit Polyethylen-kaschiertes Papier einzeln eingesiegelt und nach dem üblichen Verfahren sterilisiert (Abb. 2). Zum Nachweis der Keime im Harn wird der Teststreifen aus der Umverpackung herausgenommen, in frischen Mittelstrahlurin getaucht und in einem Bebrütungsgefäß 12 – 24 Stunden bei 37°C bebrütet. Die harnpathogenen Keime zeigen auf solchen Teststreifen das gleiche Wachstumsverhalten wie auf den entsprechend klassischen Agar-Nährmedien.

In der Abb. 1, die eine Aufsicht auf einen erfindungsgemäßen Teststreifen mit zwei Testfeldern zeigt, bedeutet (1) den Nährkarton, (2) den darüberliegenden Film und (3) die als Handgriff und Unterlage dienende Folie.

Die Abb. 2 zeigt einen Seitenriß eines einzeln eingesiegelten erfindungsgemäßen Teststreifens, der je 2 Testfelder auf der Vorder- und Rückseite aufweist. Wie in Abb. 1 ist mit (1) der Nährkarton, mit (2) der Film und mit (3) die Griffolie bezeichnet, (4) soll die Verpackung andeuten, in die der Streifen eingesiegelt ist.

Beispiel 3
Keimzahlbestimmung und Keimdifferenzierung

Die Filme, die gemäß Beispiel 1 hergestellt wurden, werden in Quadrate von 5 cm Seiten-Länge geschnitten.

In einer Petrischale werden Nährkartons der gleichen Größe gelegt und mit sterilem Wasser befeuchtet. Als Nährmedien können alle in der Mikrobiologie bekannten Nährstoff- bzw. Nährstoff-Hemmstoffkompositionen verwendet werden.

Für die Bestimmung der Keimzahl nimmt man am besten ein Universalmedium, wie Ca – So-Medium. Zur Keimdifferenzierung nimmt man ein Elektiv- oder Selektiv-Medium, wie Mac Koncey-Medium zur weitgehenden Hemmung der grampositiven Flora, Enterokokken-Selektiv-Medium nach Slanetz und Bartley, Pseudomonas-Elektiv-Medium oder Salmonellen-Selektiv-Medium nach Wilson und Blair.

Auf die befeuchteten Nährkartons legt man Filme, deren Netzmittel so gewählt sind, daß sie dem entsprechenden Nährmedium entsprechen und verteilt mit dem Spatel eine genau pipettierte Menge der Keimsuspension.

Nach einer Bebrütungszeit von 12 – 48 Stunden bei 30°C – 37°C kann man durch Auszählen der Kolonien die Keimzahlen bestimmen und durch das Wachstumsverhalten der Keime auf den verschiedenen Medien die Art identifizieren.

Beispiel 4
Diagnose von Harnweginfekten

Zur Diagnose eines Harnweginfektes wird ein Teststreifen gemäß Beispiel 1 verwendet. Das Netzmittel des Films wird wie im Beispiel 3 auf das Nährmedium abgestimmt. Dazu schneidet man Nährkartons in einer Größe von 2,5 x 2,5 cm aus, legt sie auf eine 2,5 x 10 cm große Siegelfolie und siegelt den Film mit einer Heißsiegelwalze so auf, daß er eng an dem Nährkarton anliegt. Zweckmäßigerweise nimmt man einen Nährkarton mit CLED-Medium und einen mit Mac-Koncey-Medium, die man nebeneinander anbringt. Taucht man einen solchen Teststreifen in frisch gelassenen Mittelstrahlurin und bebrütet ihn, so findet man für alle harnpathogenen Keime, das sind vor allem: Colibakterien, Enterokokken, Proteus mirabilis, Proteus vulgaris, Proteus morganii, Proteus rettgeri, Pseudomonas aeruginosa, Candida albicans, Klebsiella, Aerobacter aerogenes, Staph. aureus, Citrobacter, das gleiche Ergebnis wie mit klassischen Nähragars. Der große Vorteil des beschriebenen Testsystems gegenüber Agar ist der, daß die Teststreifen steril verpackt, über Jahre haltbar sind und nur einen Bruchteil des Lagervolumens von Agars benötigt, auch die Vernichtung durch Verbrennung ist weniger aufwendig.

Beispiel 5
Anfertigen von Antibiogrammen

Um das Resistenzverhalten der gemäß Beispiel 4 angezüchteten Keime zu testen, wird wie folgt verfahren:

Man geht in üblicher Art und Weise vor, indem man Kolonien mit der Öse abimpft, in steriler physiologischer Kochsalzlösung aufschlämmt und 0,1 ml davon weiterverarbeitet. Dazu wird ein kreisrunder Nährkarton, beispielsweise mit Müller-Hinton-Medium, mit sterilem Wasser befeuchtet, mit einem gleichgroßen Film bedeckt, die Keimsuspension mit einem Spatel darauf verteilt und Antibiotika-Testblättchen gemäß DIN 58 940 aufgelegt. Ein Andrücken der Testblättchen ist im Gegensatz zu Nähragars nicht nötig, da diese auf der feuchten Oberfläche des Teststreifens von allein haften. Nach dem Bebrüten wurde für alle harnpathogenen Keime bei allen eingesetzten Antibiotika ein mit der Bebrütung auf normalen Agar-Nährböden identisches Resistenzverhalten beobachtet.

Beispiel 6

Bestimmung der minimalen Hemm-Konzentration

62 g einer Dispersion aus dem Copolymerisat aus Vinylacetat und Vinylpropionat, 16 g Polyvinylpyrrolidon in 22 ml Wasser gelöst, 1 ml des in Beispiel 1 beschriebenen Phosphatpuffers, 1 g Polyethylensorbitan-monooleat werden homogen verrührt und mit Natronlauge auf einen pH-Wert von 7,3 eingestellt. In diese Filmrohmasse werden die Antibiotika gemäß DIN 58 940 in steigenden Mengen zugeführt und zwar jeweils 2,0 µg, 20 µg, 200 µg, 2,0 mg und 20 mg/100 g.

Die so hergestellten Rohmassen werden wie in Beispiel 2 beschrieben mit einer Schichtdicke von 350 µ auf ein Nylonnetz von ca. 60 µ Dicke, Fadenstärke 40 µ, ausgestrichen und anschließend bei 50°C getrocknet. Aus den so gewonnenen »Antibiotikafilmen« werden Teststreifen hergestellt. Dazu legt man auf eine steife Folie 2,5 x 2,5 cm große Nährkartons mit CaSo-Medium. Über die Nährkartons siegelt man die »Antibiotikafilme«. Es können entweder für jedes Antibiotikum und jede Konzentration getrennte Teststreifen hergestellt oder auf einem Teststreifen Filme mit verschiedenen Konzentrationen desselben Antibiotikums angebracht werden.

Taucht man diese Teststreifen in z. B. keimhaltige Harne oder bringt Keimsuspensionen auf die Testbezirke auf, bebrütet sie in üblicher Art und Weise, so kann man ohne Schwierigkeiten die minimale Hemm-Konzentration bestimmen, daraus die Resistenz der Keime beurteilen und das richtige therapeutische Vorgehen ableiten.

Parallel dazu durchgeführte Bestimmungen der minimalen Hemm-Konzentration nach dem klinischen Verfahren über Nährbouillons mit anschließender nephelometrischer Auswertung zeigten, daß sich aus den Ergebnissen des erfindungsgemäßen und denen des klassischen Verfahrens die gleichen therapeutischen Konsequenzen ergeben.

Beispiel 7

Bestimmung der minimalen Hemm-Konzentration von Antibiotika, die in einem anderen pH-Bereich stabiler sind als im pH-Bereich des optimalen Keimwachstums

a) Zweiter Film auf Dispersionsbasis

Es ist bekannt, daß Cephazolin im Bereich von 5,2–5,6 über längere Zeit stabil ist als im pH-Bereich von 7,0–7,5, dem pH-Bereich des optimalen Keimwachstums harnpathogener Keime. In diesem Fall werden Zweischichten-Filme hergestellt. Dazu geht man folgendermaßen vor:

Auf einen antibiotikafreien Film, gemäß Beispiel 2, wird mit einer Schichtdicke von 35 µ die Filmrohmasse, gemäß Beispiel 6, die mit Natronlauge auf einen pH-Wert von 5,2–5,6 eingestellt ist und in die steigende Mengen Cephazolin zugeführt wurde, beschichtet. Dabei werden folgende Mengen Antibiotikum pro 100 g Filmrohmasse eingerührt: 20 µg, 200 µg, 2,0 mg, 20 mg und 200 mg.

Die Weiterverarbeitung, das Trocknen, die Herstellung von Teststreifen, sowie die Testung des Resistenzverhaltens der Keime in Harnen und Keimsuspensionen, wird wie in Beispiel 6 beschrieben, durchgeführt.

Parallel dazu durchgeführte Ermittlungen der minimalen Hemm-Konzentration nach den klassischen Verfahren brachten die gleichen Ergebnisse, wie in Beispiel 6 beschrieben.

So hergestellte »Zweischichten«-Filme zeigen noch nach 12 Monaten mehr als 95% der Aktivität des enthaltenen Cephazolins, während ein Film, der Cephazolin bis pH 7,0 enthält, bereits auf unter 50% der Aktivität abgefallen ist.

b) Zweiter Film aus Filmbildnern, die keine Dispersion sind

Der zweite, antibiotikahaltige Film kann statt auf Dispersionsbasis auch nur auf Basis eines Filmbildners, der die Antibiotika während der Bebrütungsphase freigibt, aber keine Dispersion ist, aufgebaut sein.

20 g homopolymeres Ethylenoxid werden ad 100 ml in Wasser gelöst, dazu kommen 1 g Polyoxyethylensorbitanoleat, die Lösung wird auf den notwendigen pH-Wert von 5,2–5,6 mit Salzsäure eingestellt, die unter a) aufgeführten Mengen Cephazolin zugegeben und homogen verrührt.

Die Weiterverarbeitung und die mit solchen Testen erhaltenen Ergebnisse sind gleich wie bei a) beschrieben.

Beispiel 8

Teststreifen, bei denen im Film zusätzlich zu makromolekularen Öffnern Nährstoffe enthalten sind

62 g einer Dispersion aus einem Copolymerisat von Vinylacetat und Vinylpropionat werden vermischt mit 37 g einer Lösung der Zusammensetzung 37 g Polyvinylpyrrolidon, 13,0 g sec.

Natriumphosphat, 2,3 g prim. Kaliumphosphat in 500 ml Wasser gelöst und dazu 2,0 g Lactose und 1,0 g Polyoxyethylensorbitanmonooleat gerührt.

Aus der Filmrohmasse werden, wie in Beispiel 2 beschrieben, Filme hergestellt und wie dort beschrieben, Teststreifen durch Zusammenfügen mit einem Nährkarton, der mit Universalmedium getränkt ist, hergestellt.

Harnpathogene Keime zeigen auf solchen Teststreifen teilweise schöner ausgebildete Kolonien als auf klassischen Agar-Nährmedien.

Beispiel 9

Teststreifen, bei denen im Film nur Nährstoffe als Öffner eingesetzt werden

Es wird eine Filmrohmasse folgender Zusammensetzung hergestellt. Zu 100 g einer Dispersion aus einem Copolymerisat von Vinylacetat und Vinylpropionat werden 10 ml eines 0,7 m Phosphatpuffers, in dem 1,5 g Glucose und 1,5 g Lactose gelöst sind, zugeführt. Danach werden 1,0 g Polyoxyethylensobitanmonooleat eingerührt und die Filmrohmasse mit Natronlauge auf einen pH von 7,3 eingestellt.

Mit diesen Filmrohmassen werden Teststreifen in der in Beispiel 8 beschriebenen Weise hergestellt.

Von den harnpathogenen Keimen wächst auf diesen Teststreifen der Pseudomonas aeruginosa deutlich besser, als die meisten anderen, so daß man mit diesem Test eine orientierende Prüfung auf diesem Keim vornehmen kann.

**Patentansprüche**

1. Vorrichtung für mikrobiologische Arbeiten, bestehend aus einem Nährkarton und einer für Bakterien undurchlässigen, für Nährstoffe durchlässigen Abdeckschicht, dadurch gekennzeichnet, daß die Abdeckschicht aus einem wasserunlöslichen, mit Öffnern modifizierten Kunststoffilm besteht, der aus einer wäßrigen Dispersion des Kunststoffs hergestellt ist.

2. Vorrichtung gemäß Anspruch 1, dadurch gekennzeichnet, daß als Öffner eine wasserlösliche oder wasserquellbare Verbindung verwendet wird.

3. Vorrichtung gemäß den Ansprüchen 1—2, dadurch gekennzeichnet, daß Öffner und Kunststoff im Gewichtsverhältnis von 5 : 1 bis 1 : 50 enthalten sind.

4. Vorrichtung gemäß den Ansprüchen 1—3, dadurch gekennzeichnet, daß der Film ein Kunststoffnetz, vorzugsweise in einer Dicke von 30—200 μ als Trägergerüst enthält.

5. Vorrichtung gemäß den Ansprüchen 1—4, dadurch gekennzeichnet, daß die Abdeckschicht zusätzlich Netzmittel, Puffer, Salze oder Nährstoffe enthält.

6. Vorrichtung gemäß den Ansprüchen 1—5, dadurch gekennzeichnet, daß die Abdeckschicht das Wachstum bestimmter Mikroorganismen hemmende und/oder fördernde Stoffe enthält.

7. Vorrichtung gemäß den Ansprüchen 1—6, dadurch gekennzeichnet, daß der Nährkarton zusätzlich Netzmittel, Feuchthaltemittel, Quellstoffe, Puffer, Salze oder Antibiotika enthält.

8. Verfahren zur Bestimmung von Keimzahlen und zur Keimdifferenzierung unter Benutzung einer Vorrichtung gemäß den Ansprüchen 1—7, dadurch gekennzeichnet, daß man die Vorrichtung in die Untersuchungslösung eintaucht, einen Flüssigkeitsüberschuß abstreift, anschließend in üblicher Weise bebrütet und die gebildeten Bakterienkolonien auszählt bzw. durch das Wachstumsverhalten der Keime auf verschiedenen Medien die Arten differenziert.

**Claims**

1. Device for microbiological procedures, consisting of a nutrient card and a covering layer which is impermeable to bacteria but permeable to nutrients, characterised in that the covering layer consists of a water-insoluble synthetic resin film modified with openers which is produced from an aqueous dispersion of the synthetic resin.

2. Device according to claim 1, characterised in that a water-soluble or water-swellable compound is used as opener.

3. Device according to claims 1—2, characterised in that opener and synthetic resin are contained in a weight ratio of 5 : 1 to 1 : 50.

4. Device according to claims 1—3, characterised in that the film contains a synthetic resin mesh, preferably with a thickness of 30—200 μ, as carrier support.

5. Device according to claims 1—4, characterised in that the covering layer additionally contains wetting agents, buffers, salts or nutrients.

6. Device according to claims 1—5, characterised in that the covering layer contains materials which inhibit and/or promote the growth of particular micro-organisms.

7. Device according to claims 1—6, characterised in that the nutrient card additionally contains wetting agents, moisture-retaining agents, swelling agents, buffers, salts or antibiotics.

8. Method for determining microbe counts and for differentiating microbes with the use of a device according to claims 1—7, characterised in that one dips the device into the solution to be investigated, wipes off an excess of liquid, subsequently incubates in known manner and counts the colonies of bacteria formed or differentiates the types by the growth behaviour of the microbes on various media.

**Revendications**

1. Dispositif pour travaux microbiologiques comprenant un carton de culture et une couche de recouvrement imperméable aux bactéries et perméable aux substances de culture, caractérisé en ce que la couche de recouvrement est un film en une matière synthétique modifiée par des perméabilisateurs, fabriquée à partir d'une dispersion aqueuse de la matière synthétique.

2. Dispositif selon la revendication 1, caractérisé en ce que l'on utilise comme perméabilisateur une substance hydro soluble ou gonflant au contact de l'eau.

3. Dispositif selon la revendication 1 ou 2, caractérisé en ce que le rapport en poids entre le perméabilisateur et la matière synthétique est compris entre 5 : 1 et 1 : 50.

4. Dispositif selon l'une quelconque des revendications 1 à 3, caractérisé en ce que le film contient en tant que structure de support un réseau en matière synthétique dont l'épaisseur des fils est comprise entre 30 et 200 $\mu$.

5. Dispositif selon l'une quelconque des revendications 1 à 4, caractérisé en ce que la couche de recouvrement contient une addition d'agents mouillants, de tampons, de sels ou de substance de culture.

6. Dispositif selon l'une quelconque des revendications 1 à 5, caractérisé en ce que la couche de recouvrement renferme des substances favorisant ou freinant la croissance de certains micro-organismes.

7. Dispositif selon l'une quelconque des revendications 1 à 6, caractérisé en ce que le carton de culture contient une addition d'agents mouillants, d'agents pour le maintien de l'humidité, d'agents gonflants de tampons, de sels ou d'antibiotiques.

8. Procédé pour la détermination du nombre de germes et pour la différenciation des germes à l'aide du dispositif selon l'une quelconque des revendications 1 à 7, caractérisé en ce que l'on immerge le dispositif dans la solution à examiner, on essore l'excès de liquide, on incube et on compte les colonies de bactéries formées, respectivement on différencie les espèces selon le comportement de croissance des germes sur les différents milieux.

1/1

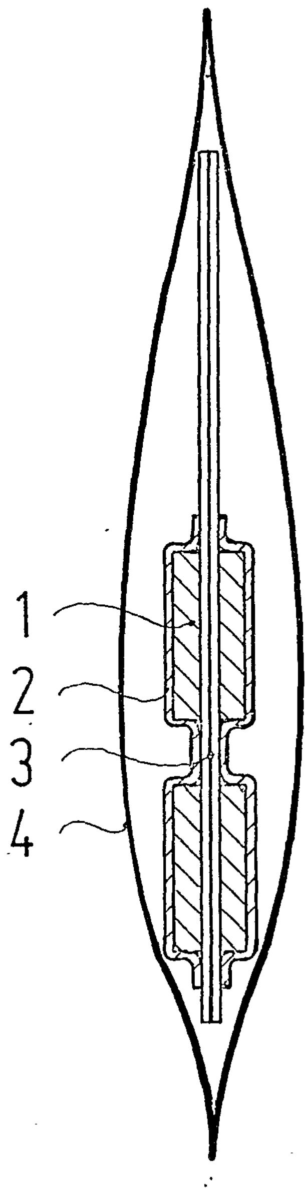


Abb. 2

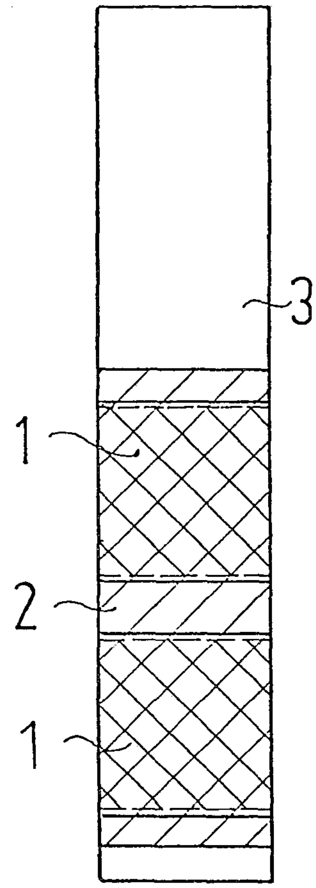


Abb. 1

1 Nährkarton
2 Film
3 Trägerfolie
4 Verpackung